# EUROPEAN PATENT APPLICATION

(11) **EP 1 715 059 A1**
(43) Date of publication of application: **25.10.2006**
(21) Application number: 05008729.5
(22) Date of filing: 21.04.2005
(51) Int. Cl.: C12Q 1/48, G01N 33/50

(54) **Method for the identification of possibly harmful receptor tyrosine kinase (RTK) mutations and of inhibitors or medication directed against RTK mutants**

(71) Applicant: Esbatech AG, 8952 Schlieren (CH)
(72) Inventor: Gunde, Tea, 8005 Zürich (CH); Barberis, Alcide, 8057 Zürich (CH); Berset, Catherine, 5210 Windisch (CH)
(74) Representative: Blum, Rudolf Emil

(57) **Abstract**

Described is a method for the identification of mutations in a receptor tyrosine kinase leading to a change in activity, said method comprises the steps of providing a host cell comprising a nucleic acid sequence derived from tissue, in particular cancerous tissue, of a particular individual said nucleic acid sequence encoding a polypeptide which comprises an active part of a receptor tyrosine kinase under the control of an inducible promoter, and determining the activity level of said RTK or rather the deviation of the activity from a standard form of the respective RTK, e.g. the wild-type (wt) RTK. Host cells prepared and characterized in the scope of the above defined method may then be used to determine the responsiveness to specific RTK inhibitors and/or to find new RTK inhibitors.

SEQUENCE LISTING

<110>  ESBATech

<120>  Method for the identification of possibly harmful receptor tyrosin kinase
(RTK) mutations and of inhibitors or medication directed against RTK mutants

<130>  12012EP

<160>  6

<170>  PatentIn version 3.1

<210>  1
<211>  463
<212>  DNA
<213>  Saccharomyces cerevisiae

<400>  1
gaattcacgg attagaagcc gccgagcggg tgacagccct ccgaaggaag actctcctcc      60
gtgcgtcctc gtcctcaccg gtcgcgttcc tgaaacgcag atgtgcctcg cgccgcactg     120
ctccgaacaa taaagattct acaatactag cttttatggt tatgaagagg aaaaattggc     180
agtaacctgg ccccacaaac cttcaaatga acgaatcaaa ttaacaacca taggatgata     240
atgcgattag ttttttagcc ttatttctgg ggtaattaat cagcgaagcg atgatttttg     300
atctattaac agatatataa atgcaaaaac tgcattaacc actttaacta atactttcaa     360
cattttcggt ttgtattact tcttattcaa atgtaataaa agtatcaaca aaaaattgtt     420
aatatacctc tatactttaa cgtcaaggag aaaaaactct aga                       463

<210>  2
<211>  445
<212>  DNA
<213>  Saccharomyces cerevisiae

<400>  2
gaattcagcg ggtgacagcc ctccgaagga agactctcct ccgtgcgtcc tcgtcctcac      60
cggtcgcgtt cctgaaacgc agatgtgcct cgcgccgcac tgctccgaac aataaagatt     120

ctacaatact agcttttatg gttatgaaga ggaaaaattg gcagtaacct ggccccacaa 180
accttcaaat gaacgaatca aattaacaac cataggatga taatgcgatt agttttttag 240
ccttatttct ggggtaatta atcagcgaag cgatgatttt tgatctatta acagatatat 300
aaatgcaaaa actgcattaa ccactttaac taatactttc aacattttcg gtttgtatta 360
cttcttattc aaatgtaata aaagtatcaa caaaaaattg ttaatatacc tctatacttt 420
aacgtcaagg agaaaaaact ctaga 445

<210> 3
<211> 429
<212> DNA
<213> Saccharomyces cerevisiae

<400> 3
gaattccgga ggactgtcct ccgaaattcc gtcctcgtcc tcaccggtcg cgttcctgaa 60
acgcagatgt gcctcgcgcc gcactgctcc gaacaataaa gattctacaa tactagcttt 120
tatggttatg aagaggaaaa attggcagta acctggcccc acaaaccttc aaatgaacga 180
atcaaattaa caaccatagg atgataatgc gattagtttt ttagccttat ttctggggta 240
attaatcagc gaagcgatga tttttgatct attaacagat atataaatgc aaaaactgca 300
ttaaccactt taactaatac tttcaacatt ttcggtttgt attacttctt attcaaatgt 360
aataaaagta tcaacaaaaa attgttaata tacctctata ctttaacgtc aaggagaaaa 420
aactctaga 429

<210> 4
<211> 426
<212> DNA
<213> Saccharomyces cerevisiae

<400> 4
gaattcaagg aagactctcc tccgtgcgtc ctcgtcctca ccggtcgcgt tcctgaaacg 60
cagatgtgcc tcgcgccgca ctgctccgaa caataaagat tctacaatac tagcttttat 120
ggttatgaag aggaaaaatt ggcagtaacc tggccccaca aaccttcaaa tgaacgaatc 180
aaattaacaa ccataggatg ataatgcgat tagtttttta gccttatttc tggggtaatt 240
aatcagcgaa gcgatgattt ttgatctatt aacagatata taaatgcaaa aactgcatta 300
accactttaa ctaatacttt caacattttc ggtttgtatt acttcttatt caaatgtaat 360
aaaagtatca acaaaaaatt gttaatatac ctctatactt taacgtcaag gagaaaaaac 420
tctaga 426

<210> 5

<211> 429

<212> DNA

<213> Saccharomyces cerevisiae

<400> 5
gaattccggg tgacagccct ccgaaattcc gtcctcgtcc tcaccggtcg cgttcctgaa 60

acgcagatgt gcctcgcgcc gcactgctcc gaacaataaa gattctacaa tactagcttt 120

tatggttatg aagaggaaaa attggcagta acctggcccc acaaaccttc aaatgaacga 180

atcaaattaa caaccatagg atgataatgc gattagtttt ttagccttat ttctggggta 240

attaatcagc gaagcgatga tttttgatct attaacagat atataaatgc aaaaactgca 300

ttaaccactt taactaatac tttcaacatt ttcggtttgt attacttctt attcaaatgt 360

aataaaagta tcaacaaaaa attgttaata tacctctata ctttaacgtc aaggagaaaa 420

aactctaga 429

<210> 6

<211> 406

<212> DNA

<213> Saccharomyces cerevisiae

<400> 6
gaattccgtc ctcgtcctca ccggtcgcgt tcctgaaacg cagatgtgcc tcgcgccgca 60

ctgctccgaa caataaagat tctacaatac tagcttttat ggttatgaag aggaaaaatt 120

ggcagtaacc tggccccaca aaccttcaaa tgaacgaatc aaattaacaa ccataggatg 180

ataatgcgat tagtttttta gccttatttc tggggtaatt aatcagcgaa gcgatgattt 240

ttgatctatt aacagatata taaatgcaaa aactgcatta accactttaa ctaatacttt 300

caacattttc ggtttgtatt acttcttatt caaatgtaat aaaagtatca acaaaaaatt 360

gttaatatac ctctatactt taacgtcaag gagaaaaaac tctaga 406

## Description

### Technical Field

The present application relates to a cell-based method for the identification of abnormal receptor tyrosine kinase (RTK) activity and/or for the identification of inhibitors suitable to inhibit receptor tyrosine kinase activity also or especially of RTKs with specific abnormal activity.

### Background Art

Receptor tyrosine kinases (RTKs) are key regulators of intercellular communication that controls cell growth, proliferation, differentiation, survival and metabolism. About 20 different RTK families have been identified that share a similar structure, namely an extracellular binding site for ligands, a transmembrane region and an intracellular tyrosine kinase domain (1). Extracellular ligand binding induces or stabilizes receptor dimerization leading to increased RTK kinase activity. The intracellular catalytic domain displays the highest level of conservation among RTKs and includes the ATP-binding site that catalyzes receptor autophosphorylation of cytoplasmic tyrosine residues, which serve as docking sites for Src homology 2 (SH2)-and phosphotyrosine-binding (PTB) domain-containing proteins such as Grb2, Shc, Src, Cbl or phospholipase C y.These proteins subsequently recruit additional effectors containing SH2, SH3, PTB and pleckstrin-homology (PH) domains to the activated receptor, which results in the assembly of signaling complexes at the membrane and the activation of a cascade of intracellular biochemical signals. The most important downstream signaling cascades activated by RTKs include the Ras-extracellular regulated kinase (ERK)-mitogen activated (MAP) kinase pathway, the phosphoinositide 3-kinase (PI 3-kinase)-Akt and the JAK/STAT pathway. The complex signaling network triggered by RTKs eventually leads either to activation or repression of various subsets of genes and thus defines the biological response to a given signal.

The activity of RTKs and their mediated cellular signaling is precisely coordinated and tightly controlled in normal cells. Deregulation of the RTK signaling system, either by abnormal stimulation through growth factors and/or through genetic alteration, result in deregulated tyrosine kinase activity. These aberrations generally result in RTKs with constitutive or strongly enhanced kinase activity and subsequent signaling capacity, which leads to malignant transformation. Therefore, they are frequently linked to human cancer and also to other hyperproliferative diseases such as psoriasis (2). The most important mechanisms leading to constitutive RTK signaling include overexpression and/or gene amplification of RTKs, genetic alterations such as deletions and mutations within the extracellular domain as well as alterations of the catalytic site, or autocrine-paracrine stimulation through aberrant growth factor loops.

For example, in many human cancers, gene amplification and/or overexpression of RTKs occurs, which might increase the response of cancer cells to normal growth factor levels. Additionally, overexpression of a specific RTK on the cell surface increases the incidence of receptor dimerization even in the absence of an activating ligand. In many cases this results in constitutive activation of the RTK leading to aberrant and uncontrolled cell proliferation and tumor formation. An important example for such a scenario is HER2, also known as ErbB2, that belongs to the epidermal growth factor (EGF) receptor family of RTKs. Overexpression of HER2 was found in various types of human cancers, especially in human breast and ovarian carcinomas (3). Most importantly, aberrantly elevated levels of HER2 correlate with more aggressive progression of disease and reduced patient survival time (4). EGFR, which was the first receptor tyrosine kinase to be molecularly cloned (5), also plays a fundamental role in tumorigenesis. EGFR is frequently overexpressed in non-small-cell lung, bladder, cervical, ovarian, kidney and pancreatic cancer and in squamous-cell carcinomas of the head and neck (6). The predominant mechanism leading to EGFR overexpression is gene amplification with up to 60 copies per cell reported in certain tumors (7). In general, elevated levels of EGFR expression are associated with high metastatic rate and increased tumor proliferation (8).

Since tyrosine kinases have been implicated in a variety of cancer indications, RTKs and the activated signaling cascades represent promising areas for the development of target-selective anticancer drugs. One approach to inhibit aberrant RTK signaling is the development of small-molecule drugs that selectively interfere with their intrinsic tyrosine kinase activity and thereby block receptor autophosphorylation and activation of downstream signal transducers (9).

Several methods have been developed to screen compound libraries in order to identify RTK-specific inhibitors, most of which utilize biochemical assays (10). One important aspect to consider for the selection of effective tyrosine kinase inhibitors is that these compounds must be able to permeate through cellular membranes and function in an intracellular environment for the necessary period of time. In addition, in order to become potential drug candidates kinase inhibitors must not show general and non-specific cytotoxic effects that might also kill healthy cells. It is therefore desirable to have a cellular system for the primary screening of compounds capable of inhibiting RTK activity. The requirements for such *in vivo* assays are the ability to examine a specific cellular process triggered by a defined target and a means to readily measure its output in a high-throughput screening system (HTS). The availability of an increasing number of biotechnological tools to genetically modify cells and microorganisms have allowed the development of simple read-out assays for cellular processes that can be readily applied to automated systems in HTS (11-14). Cellular screens should ideally be performed with cells of human origin, which evidently provide the most physiologically relevant model system. However, the effects of redundant processes on the measured output can be difficult to control and to distinguish from the effects that are expected to be specific for the defined target; and genetic manipulation of mammalian cells is generally problematic and time-consuming. Moreover, human cells are expensive to culture and sometimes difficult to propagate in automated systems used for HTS. Microorganisms such as yeast present a convenient alternative for measuring the activity of defined human proteins in a heterologous, yet cellular (eukaryotic) environment. In yeast cells, the function of human proteins can often be reconstituted and aspects of some human physiological processes can be recapitulated because of the high degree of conservation of basic molecular and cellular mechanisms between yeast and human cells (14-17). The fact that many human proteins function in yeast indicates that the required conformation, stability, protein-protein interaction, etc. are taking place in this eukaryotic organism.

Some RTK inhibitors are already known, and a reliable cell-based method for the identification and/or validation of new inhibitors of a receptor tyrosine kinase, which permeate cell membranes and which are not cytotoxic, is described in co-pending international application no. PCT/CH03/00694 filed October 24, 2003. There is, however, evidence suggesting that such inhibitors are not effective in all patients or at least not identically effective. It has e.g. been shown by Paez et al. (20), Lynch et al. (21), and Green (22) that there is a correlation between EGFR mutations and the response to Gefitinib therapy. Corbin et al. (23) found a correlation between oncogenic KIT mutants and the sensitivity to the kinase inhibitors MLN518 and PD180970. All these studies have been performed by either comparing the DNA sequences of persons without cancer, patients with cancer that responded to a specific therapy and of patients who did not respond to a specific therapy, and/or by using carcinogenic tumor derived human cell lines.

In view of these findings showing that a successful therapy may be dependent on the disease causing mutation, a need for a method allowing the easy identification of possibly disease causing mutations in RTKs and of drugs suitable for the therapy of respective mutation induced diseases exists.

In vitro kinase assays may not be optimal for some of said kinases since they do not sufficiently mimic the intracellular environment. Moreover, for the purpose of functionally screening a large number of RTK sequences, e.g. from cancer patient biopsies, biochemical in vitro assays would be labor-intensive and time consuming, and thus inexpedient, since each isolated RTK would have to be expressed and purified, e.g. from E. coli, for measuring its activity and inhibition by drug compounds in vitro. In the last few years, cell-based assays have become an increasingly attractive alternative to in vitro biochemical assays for high-throughput-screening. A rapid yeast based assay for the identification of modulators of RTK activity has been developed (24). Said assay is based on an inducible promoter, specifically a promoter that is inactive in the presence of glucose and active in the presence of galactose. The positive read-out for RTK inhibition measure as cell growth avoids selection of toxic compounds because they inhibit cell proliferation per se.

Although this cell based assay is suitable for high-throughput-screening, it is not suitable for identifying carcinogenic mutations. Thus, there is still a need for such an assay allowing the easy identification of such mutations.

### Disclosure of the Invention

Hence, it is one object of the invention to provide a method for the identification of mutations in a receptor tyrosine kinase leading to a change in activity, in particular leading to an enhanced activity. Said method comprises the following steps (all steps being performed outside/ independent from the human or mammalian body):
a) providing a nucleic acid construct, said nucleic acid construct comprising under the control of an inducible promoter a nucleic acid sequence of a particular individual encoding a polypeptide which comprises a tyrosine kinase domain of a receptor tyrosine kinase or a fragment of said tyrosine kinase domain wherein said polypeptide lacks a transmembrane domain or a functional fragment of said transmembrane domain,
b) introducing said nucleic acid construct into a host cell and
c) culturing said cells under conditions in which the tyrosine kinase activity of an active RTK leads to reduced or arrested proliferation of said host cells,
d) determining the activity level of said RTK, and
e) comparing the activity of said RTK with the activity determined for a standard form of the respective RTK under the same conditions.

It is a further object of the invention to provide a method for the identification and/or validation of inhibitors of a specific receptor tyrosine kinase activity. Said method comprises the following steps:
a) providing host cells comprising a nucleic acid construct of a particular individual tyrosine kinase activity under the control of an inducible promoter, said nucleic acid construct encoding a polypeptide which comprises a tyrosine kinase domain of a receptor tyrosine kinase or a functional fragment thereof wherein said polypeptide lacks a transmembrane domain or a functional fragment of said transmembrane domain and wherein said tyrosine kinase activity leads to reduced or arrested proliferation of said host cells,
b) contacting said host cells with at least one candidate compound and
c) determining the inhibitory effect of such at least one candidate compound on said tyrosine kinase activity by cultivation of said host cells under suitable conditions such that a modulation of the tyrosine kinase activity by said at least one candidate compound leads to cell proliferation or enhanced cell proliferation.

The term a "particular individual" as it is used herein refers to one given person, in particular a given patient if the RTK is derived from a tumor or a blood sample in the case of leukemia.

The "standard form of the respective RTK" in general will be the wild-type form of the respective RTK, i.e. the same type of RTK; however, it may be any form having a suitable basal activity, e.g. also the same RTK found in healthy tissue or blood of the same individual.

In order to get viable cell lines, the nucleic acid construct of a specific individual tyrosine kinase activity is under the control of an inducible promoter which controls the expression of said polypeptide, preferably a promoter that is inactivated in the presence of glucose and activated in the presence of galactose. Promoters of such type are e.g. the GAL1 promoter or promoters derived thereof that due to stepwise deletions lead to different expression levels (see below). Promoters leading to different expression levels are advantageously used in cells/cell lines prepared for the identification of mutations in a receptor tyrosine kinase leading to a change in activity, since they allow the determination of enhanced/reduced activity within suitable time.

In a preferred embodiment of the present invention said nucleic acid construct encodes a polypeptide which comprises the entire cytoplasmic part of said receptor tyrosine kinase.

In a further preferred embodiment said polypeptide lacks signal sequences and/or peptides leading to nuclear localization and/or to membrane anchoring of said polypeptide.

In a further preferred embodiment said polypeptide further comprises a dimerization domain or a functional fragment thereof. Said dimerization domain is preferably selected from c-Fos leucine zipper, c-Jun leucine zipper and Gcn4 leucine zipper.

In a much preferred embodiment the host cells comprise two polypeptides wherein the first polypeptide comprises a c-Fos leucine zipper and the second polypeptide comprises a c-Jun leucine zipper.

In another preferred embodiment said polypeptide comprises a sequence leading to membrane anchoring of the polypeptide, in particular a myristolation or a farnesylation signal for membrane anchoring.

The tyrosine kinase activity for use in the method of the present invention can stem from any receptor tyrosine kinase. Preferred receptor tyrosine kinases are EGFR, ERBB2, ERBB3, ERBB4, INSR, IGF-1R, IRR, PDGFRα, PDGFRβ, CSF-1R, KIT/SCFR, FLK2/FLT3, VEGFR 1-3, FGFR 1-4, CCK4, TRKA, TRKB, TRKC, MET, RON, EPHA 1-8, EPHB 1-6, AXL, MER, TYRO3, TIE, TEK, RYK, DDR1, DDR2, RET, ROS, LTK, ALK, ROR1, ROR2, MUSK, AATYK, AATYK2, AATYK3, RTK 106.

In a much preferred embodiment said tyrosine kinase activity is selected from the group consisting of INSR, IGF-1R, PDGFRα, PDGFRβ, KIT/SCFR, TRKA, MET, RON, EPHB2, EPHB4, AXL, TEK, RET, ROS, CSF-1R, FLK2/FLT3, TRKB, TRKC, EPHA 2, TIE, ALK, EGFR, ERBB2, VEGFR 1, VEGFR 2, FGFR 1-4.

In another preferred embodiment said receptor tyrosine kinase has human origin.

In yet another preferred embodiment said host cells are yeast cells or bacterial cells, preferably S. cerevisiae cells, more preferably S. cerevisiae cells harboring a mutation in one gene or any combination of genes selected from the group comprising the ABC transporter genes, the ERG6 gene and genes encoding phosphatases, in particular the PTP1 gene.

It is a further aspect of the present invention to provide an RTK data base wherein the activities found are correlated with the nucleic acid sequence and/or the amino acid sequence, and/or the mutations found in comparison to the wild-type sequence. The sequences may also be and preferably are correlated with the results found for specific inhibitors tested. This enables the fast selection of the presumably best therapy.

### Brief Description of the Drawings

The invention will be better understood and objects other than those set forth above will become apparent when consideration is given to the following detailed description thereof. Such description makes reference to the annexed drawings, wherein:
Figure 1a shows three c-Met constructs and the effect of their expression on yeast growth;
Figure 1b shows three PDGFRβ constructs and the effect of their expression on yeast growth. Glucose = repressive conditions i.e. the polypeptides are not expressed and galactose = permissive conditions i.e. polypeptides are expressed;
Figure 2a shows a Western blot analysis of cell extracts with anti-myc antibodies;
Figure 2b shows a Western blot analysis of cell extracts with anti-phosphotyrosine antibodies and
Figure 3 shows the inhibition of the Receptor Tyrosine Kinase activity of PDGFRβ by Gleevec in yeast cells.
Figure 4 shows the sequence of the GAL1 promoter with Gal4 binding sites (BS I to BS IV having a consensus sequence of 17bp length) marked as well as the Mig 1 binding sites, TATA box, restriction sites and transcription start site indicated.
Figure 5 shows the deletions leading to reduced protein expression.
Figure 6 shows the growth of yeast strains expressing various levels of RET and EPHB4, respectively, after different times.
Figure 7a shows the growth of yeast strains expressing wild-type KIT as well as different KIT mutants, namely D816V, K642E, and N822H.
Figure 7b shows the growth of yeast strains expressing wild-type KIT as well as different KIT mutants, namely V560G, D816Y, and D816F.
Figure 8a shows the sensitivity of wild-type (wt) KIT and the KIT mutants V560G and K642E towards 3 inhibitors.
Figure 8b shows the sensitivity of wt KIT and the KIT mutants D816V and D816Y towards 3 inhibitors.
Figure 8c shows the sensitivity of wt KIT and the KIT mutants D816F and N822H towards 3 inhibitors.
Figure 9 shows that expression of EGFR(L858R) in *S. cerevisiae* causes a kinase-dependent inhibition of growth.
Figure 10 shows a Western blot analysis of wild-type EGFR and EGFR mutants in ΔPTP1 yeast strains.

### Modes for Carrying Out the Invention

The present invention provides a cell-based system for - in a first aspect - the identification of mutations in a receptor tyrosine kinase leading to a change in activity, in particular an enhanced activity. The nucleic acid construct encoding a receptor tyrosine kinase of interest in general is obtained from a biopsy of a particular individual, in particular a biopsy taken from a tumor, especially a biopsy of cancerous tissue.

The term biopsy as it is used herein comprises tissue samples or liquid samples, e.g. blood samples.

The method of the present invention allows to compare the activity of RTKs obtained from tumor biopsies (or from blood of patients with leukemia) with RTKs obtained from healthy tissue or healthy blood of the same individual and/or with a standard form of the respective RTKs.

A preferred method to get the desired nucleic acid construct is by means of reverse transcriptase PCR (RTPCR). The method for the identification of specific mutations in RTKs comprises the following steps performed independent of a human or mammalian body:
a) providing a nucleic acid construct, said nucleic acid construct comprising under the control of a inducible promoter a nucleic acid sequence of a particular individual encoding a polypeptide which comprises a tyrosine kinase domain of a receptor tyrosine kinase or a fragment of said tyrosine kinase domain wherein said polypeptide lacks a transmembrane domain or a functional fragment of said transmembrane domain,
b) introducing said nucleic acid construct into a host cell and
c) culturing said cells under conditions in which the tyrosine kinase activity of an active RTK leads to proliferation reduction or proliferation arrest of said host cells,
d) determining the activity of said RTK, and
e) comparing the activity of said RTK with the activity determined for a standard form of the respective RTK under the same conditions.

The nucleic acid sequence of a particular individual encoding a polypeptide which comprises a tyrosine kinase domain of a receptor tyrosine kinase or a fragment thereof preferably is a tumor derived nucleic acid sequence, in particular a cancerous tumor derived nucleic acid sequence.

The cell lines obtained by the above described method or already existing cell lines may be used in the below described inventive method for the identification and/or validation of inhibitors of a specific receptor tyrosine kinase activity. Said method comprises the following steps:
a) providing host cells comprising a nucleic acid construct of a particular individual tyrosine kinase activity under the control of an inducible promoter, said nucleic acid construct encoding a polypeptide which comprises a tyrosine kinase domain of a receptor tyrosine kinase or a functional fragment thereof wherein said polypeptide lacks a transmembrane domain or a functional fragment of said transmembrane domain and wherein said tyrosine kinase activity leads to reduced or arrested proliferation of said host cells,
b) contacting said host cells with at least one candidate compound and
c) determining the inhibiory effect of such at least one candidate compound on said tyrosine kinase activity by cultivation of said host cells under suitable conditions such that a modulation of the tyrosine kinase activity by said at least one candidate compound leads to cell proliferation or enhanced cell proliferation.

Thus, the present invention provides a cell-based system for the identification of possibly disease causing mutations in RTKs and for the determination of the responsiveness of a particular individual to a specific drug and for the screening of new inhibitors to which the carriers of specific mutations or the carriers of a specific group of mutations respond.

In one aspect of the inventive system, host cells, preferably yeast cells, conditionally expressing defined fragments of RTKs, are enabled to grow in the absence of RTK expression, and to stop or reduce proliferation if expression of active RTK is induced. Since malignant mutations of RTKs may have largely enhanced activity if compared with the respective wild-type RTK, it may be advantageous to bring nucleic acid sequences encoding such highly active RTKs or fragments thereof, respectively, under the control of a less active promoter as described below.

In a second aspect of the inventive system, host cells, for example cells selected as described above, in particular yeast cells, or host cells e.g. comprising a specific RTK mutation under the control of an other inducible promoter than the one chosen for the above described selection method, are enabled to grow only upon inhibition or inactivation of the protein kinase activity. Such a positive readout allows selection of specific kinase inhibitors that must also be soluble, stable and non-cytotoxic.

In the cell-based system of the present invention, a tyrosine kinase domain of a RTK or functional fragments thereof, preferably cytoplasmic domains of RTKs or fragments thereof, are expressed in the cytoplasm of host cells either as such or as fusion polypeptides with protein sequences that are known to form strong dimers. According to the present invention, the expression of these proteins in cells, preferably yeast cells, is controlled by inducible promoters such as that of the *GAL1* gene or promoters derived thereof.

For the identification of inhibitors that selectively target RTKs, high RTK expression under expression inducing conditions is desired to enhance the sensitivity of the assay and thus to allow better quantification of the activity of the inhibitors.

For the determination of the activity of RTK mutants, promoters with reduced protein expression capacity may be preferred. Such promoters may improve the quantification of the activity of a specific mutant in comparison to a standard, e.g. the wild-type sequence. For example, the promoter may be selected such that the expression of the wild-type RTK produces a mild slow growth phenotype and expression of hyperactive mutants produces a more dramatic slow growth phenotype.

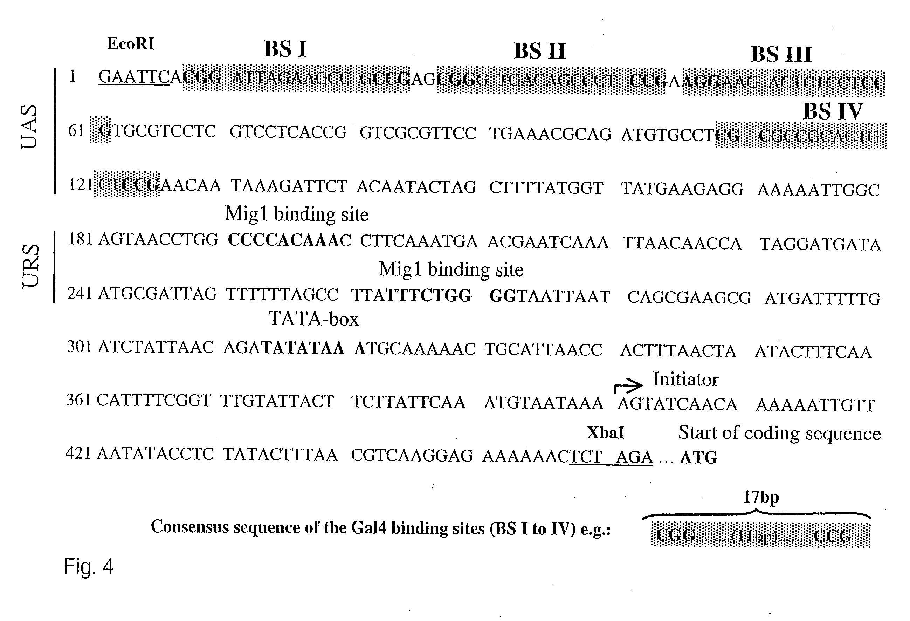

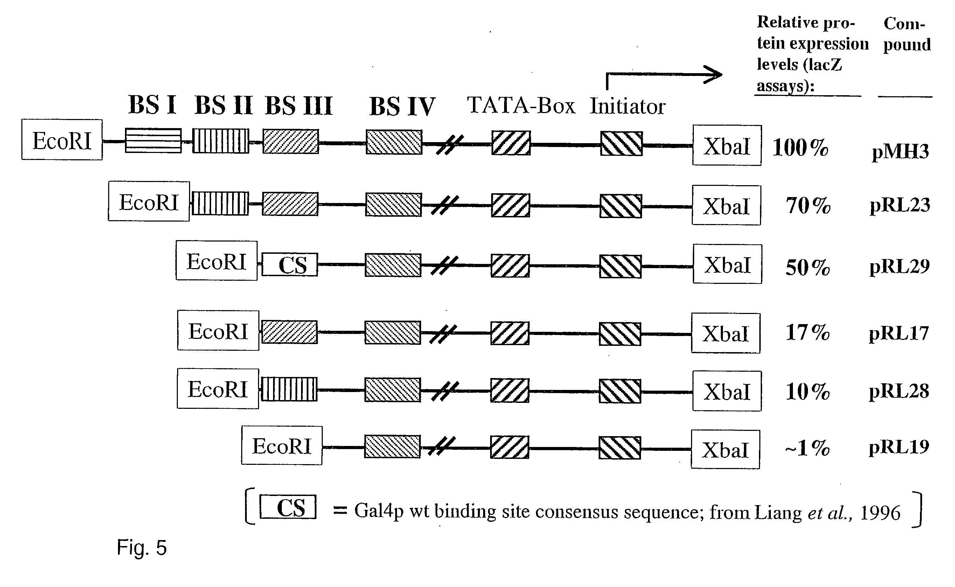

Suitable promoters with reduced protein expression are obtainable by stepwise deletions of the galactose-inducible GAL1 promoter. The GAL1 sequence with specific binding sites marked (Gal4 binding sites identified as BS I to BS IV) is shown in Figure 4 and the specific deletions leading to reduced expression levels are schematically represented in Figure 5.

The marked zones in Figure 4 have the following meaning:

Gray-shaded = Gal4 binding sites (BS in Figures 4 and 5). All natural BS I, II, III, and IV, which form the so-called UAS, are present at their natural locations on the full GAL1 promoter that show a relative expression level of 100% (pMH3 is the protein expression plasmid that bears the full GAL1 promoter).

Underlined at the 5' and 3' end are the EcoRI and XbaI restriction sites, respectively.

Underlined in the middle are the two Mig1 binding sites forming the so-called URS.

Bold = the TATA box.

**Á** = designates the A at the transcription start site.

Gray-shaded and bold = Gal4 consensus site (CS) according to Liang et al., 1996 (37).

Italic sequence = foreign sequence introduced to link either the CS (pRL29) or the BS II (pRL28) to the GAL1 promoter fragment bearing the BS IV and downstream sequences.

pMH3, 100% (SEQ. ID. NO. 1):

GAATTCACGGATTAGAAGCCGCCGAGCGGGTGACAGCCCTCCGAAGGAAGACTCTCC
TCCGTGCGTCCTCGTCCTCACCGGTCGCGTTCCTGAAACGCAGATGTGCCTCGCGCC
GCACTGCTCCGAACAATAAAGATTCTACAATACTAGCTTTTATGGTTATGAAGAGGA
AAAATTGGCAGTAACCTGGCCCCACAAACCTTCAAATGAACGAATCAAATTAACAAC
CATAGGATGATAATGCGATTAGTTTTTTAGCCTTATTTCTGGGGTAATTAATCAGCG
AAGCGATGATTTTTGATCTATTAACAGATATATAAATGCAAAAACTGCATTAACCAC
TTTAACTAATACTTTCAACATTTTCGGTTTGTATTACTTCTTATTCAAATGTAATAA
AÁGTATCAACAAAAAATTGTTAATATACCTCTATACTTTAACGTCAAGGAGAAAAAA
CTCTAGA

pRL23, 70% (SEQ. ID. NO. 2):

GAATTCAGCGGGTGACAGCCCTCCGAAGGAAGACTCTCCTCCGTGCGTCCTCGTCCT
CACCGGTCGCGTTCCTGAAACGCAGATGTGCCTCGCGCCGCACTGCTCCGAACAATA
AAGATTCTACAATACTAGCTTTTATGGTTATGAAGAGGAAAAATTGGCAGTAACCTG
GCCCCACAAACCTTCAAATGAACGAATCAAATTAACAACCATAGGATGATAATGCGA
TTAGTTTTTTAGCCTTATTTCTGGGGTAATTAATCAGCGAAGCGATGATTTTTGATC

TATTAACAGATATATAAATGCAAAAACTGCATTAACCACTTTAACTAATACTTTCAA
CATTTTCGGTTTGTATTACTTCTTATTCAAATGTAATAAAÁGTATCAACAAAAAATT
GTTAATATACCTCTATACTTTAACGTCAAGGAGAAAAAACTCTAGA

pRL29, 50% (SEQ. ID. NO. 3):

GAATTCCGGAGGACTGTCCTCCGAAATTCCGTCCTCGTCCTCACCGGTCGCGTTCCT
GAAACGCAGATGTGCCTCGCGCCGCACTGCTCCGAACAATAAAGATTCTACAATACT
AGCTTTTATGGTTATGAAGAGGAAAAATTGGCAGTAACCTGGCCCCACAAACCTTCA
AATGAACGAATCAAATTAACAACCATAGGATGATAATGCGATTAGTTTTTTAGCCTT
ATTTCTGGGGTAATTAATCAGCGAAGCGATGATTTTTGATCTATTAACAGATATATA
AATGCAAAAACTGCATTAACCACTTTAACTAATACTTTCAACATTTTCGGTTTGTAT
TACTTCTTATTCAAATGTAATAAAÁGTATCAACAAAAAATTGTTAATATACCTCTAT
ACTTTAACGTCAAGGAGAAAAAACTCTAGA

pRL17, 17% (SEQ. ID. NO. 4):

GAATTCAAGGAAGACTCTCCTCCGTGCGTCCTCGTCCTCACCGGTCGCGTTCCTGAA
ACGCAGATGTGCCTCGCGCCGCACTGCTCCGAACAATAAAGATTCTACAATACTAGC
TTTTATGGTTATGAAGAGGAAAAATTGGCAGTAACCTGGCCCCACAAACCTTCAAAT
GAACGAATCAAATTAACAACCATAGGATGATAATGCGATTAGTTTTTAGCCTTATT
TCTGGGGTAATTAATCAGCGAAGCGATGATTTTTGATCTATTAACAGATATATAAAT
GCAAAAACTGCATTAACCACTTTAACTAATACTTTCAACATTTTCGGTTTGTATTAC
TTCTTATTCAAATGTAATAAAÁGTATCAACAAAAAATTGTTAATATACCTCTATACT
TTAACGTCAAGGAGAAAAACTCTAGA

pRL28, 10% (SEQ. ID. NO. 5):

GAATTCCGGGTGACAGCCCTCCGAAATTCCGTCCTCGTCCTCACCGGTCGCGTTCCT
GAAACGCAGATGTGCCTCGCGCCGCACTGCTCCGAACAATAAAGATTCTACAATACT
AGCTTTTATGGTTATGAAGAGGAAAAATTGGCAGTAACCTGGCCCCACAAACCTTCA
AATGAACGAATCAAATTAACAACCATAGGATGATAATGCGATTAGTTTTTTAGCCTT
ATTTCTGGGGTAATTAATCAGCGAAGCGATGATTTTTGATCTATTAACAGATATATA
AATGCAAAAACTGCATTAACCACTTTAACTAATACTTTCAACATTTTCGGTTTGTAT
TACTTCTTATTCAAATGTAATAAAÁGTATCAACAAAAAATTGTTAATATACCTCTAT
ACTTTAACGTCAAGGAGAAAAAACTCTAGA

pRL19, 1% (SEQ. ID. NO. 6):

GAATTCCGTCCTCGTCCTCACCGGTCGCGTTCCTGAAACGCAGATGTGCCTCGCGCC
GCACTGCTCCGAACAATAAAGATTCTACAATACTAGCTTTTATGGTTATGAAGAGGA
AAAATTGGCAGTAACCTGGCCCCACAAACCTTCAAATGAACGAATCAAATTAACAAC
CATAGGATGATAATGCGATTAGTTTTTTAGCCTTATTTCTGGGGTAATTAATCAGCG
AAGCGATGATTTTTGATCTATTAACAGATATATAAATGCAAAAACTGCATTAACCAC
TTTAACTAATACTTTCAACATTTTCGGTTTGTATTACTTCTTATTCAAATGTAATAA
AAGTATCAACAAAAAATTGTTAATATACCTCTATACTTTAACGTCAAGGAGAAAAAA
CTCTAGA

Dependent on the activity level expected for a specific mutated RTK a promoter with reduced protein expression level, e.g. only about 70 % or 50 % or 17 % expression is used to ensure that differences are detectable within the desired time. For the identification of a candidate inhibitor the specific RTK mutant may be used under the control of the promoter leading to reduced expression level, or it may be brought under the control of a higher expressing promoter, e.g. the GAL1 promoter without deletions.

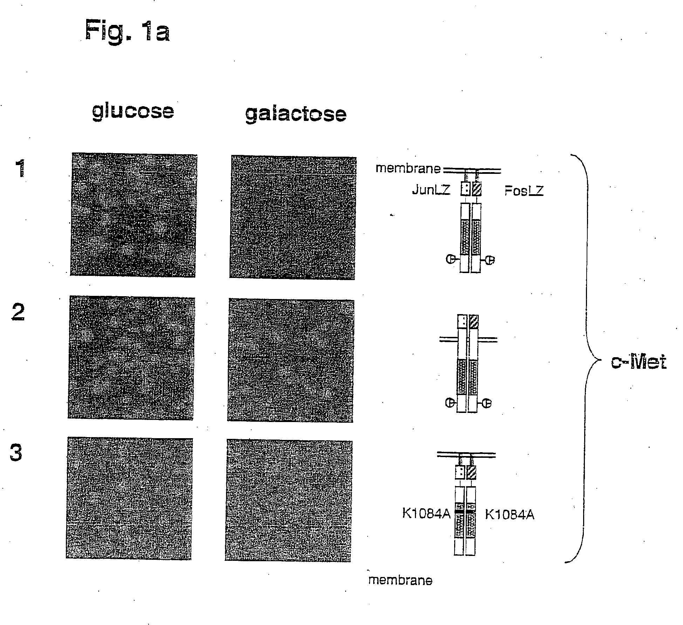

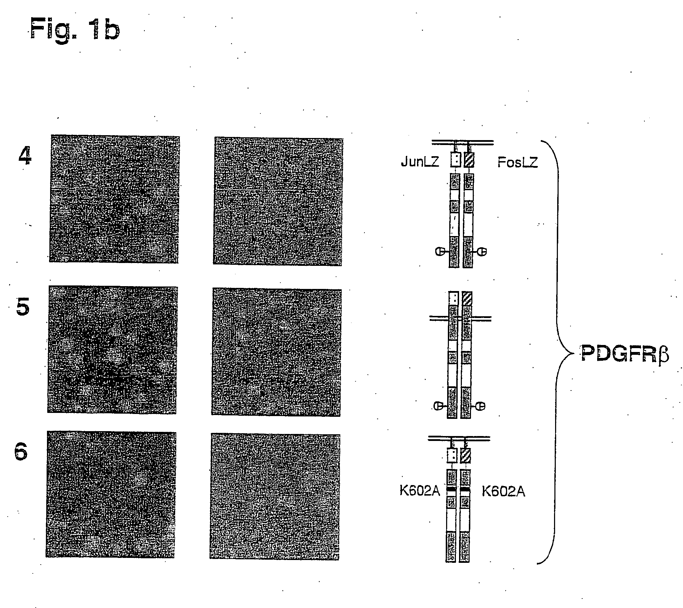

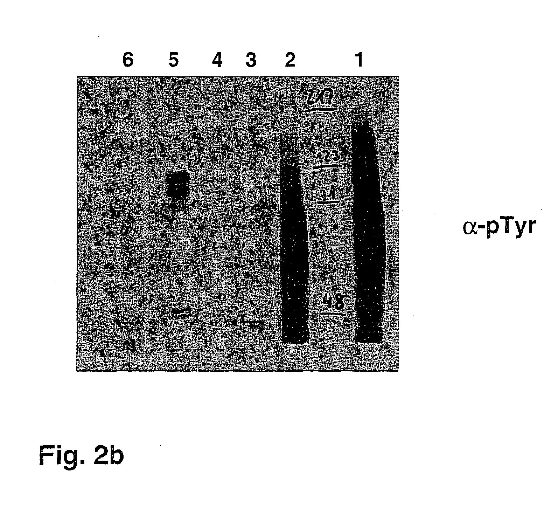

Expression of dimerizing RTK derivatives significantly reduces growth of yeast cells under selective conditions (Figures 1a and 1b). Such growth inhibition is due to the dimerization dependent activation of the tyrosine kinase activity. The introduction of a point mutation in the active site, which abolishes the tyrosine kinase function of RTKs, eliminates the growth inhibition effect (Figures 1a and 1b). Western blot analysis of cell extracts with anti-phosphotyrosine antibodies shows that the growth inhibition effect of RTKs, such as for example c-Met and PDGFRβ, correlated with its ability to catalyze tyrosine phosphorylation (Figure 2b).

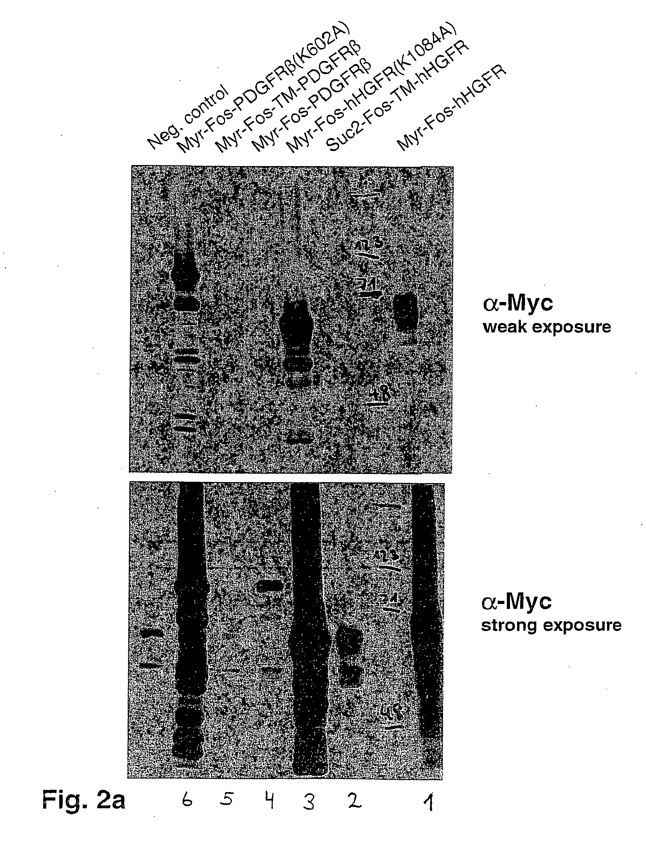

Expression of RTK polypeptides that include a transmembrane domain or a functional fragment thereof, in particular a transmembrane domain of a RTK, and a signal peptide for localization of the protein N-terminus into the ER lumen and extracellular space completely abolishes the growth inhibition effect (Figure 1a and 1b), while it does not diminish the ability of the transmembrane RTK to catalyze tyrosine phosphorylation (Figure 2b). Targeting active RTK cytoplasmic domains to the cytosolic part of cellular membranes is not relevant for the tyrosine phosphorylation and growth inhibition effects, since no difference is observed between expression of plain RTK cytoplasmic domains lacking a membrane anchoring signal and the same sequences carrying the myristolation signal for membrane anchoring (for the latter one see Figures 1a, 1b and 2a). Another possibility for membrane attachment can be farnesylation.

The prior art discloses that expression of the full-length cytoplasmic tyrosine kinase c-src in S. pombe leads to cell death (18) and that expression of pp60v-src in S.cerevisiae leads to growth arrest (19). pp60v-src mutants lacking a functional N-terminal myristolation signal cause only a partial inhibition of growth arrest (19) i.e. a complete growth arrest induced by pp60v-src expression in S. cerevisiae can only be observed when pp60v-src is targeted to its natural cell compartment. The inventors of the present invention have in contrast to the prior art found that expression of a full length RTK (comprising a transmembrane domain)in yeast cells does not lead to growth arrest i.e. targeting of a RTK to its natural cell compartment (transmembrane localisation) in yeast cells does not lead to growth arrest.

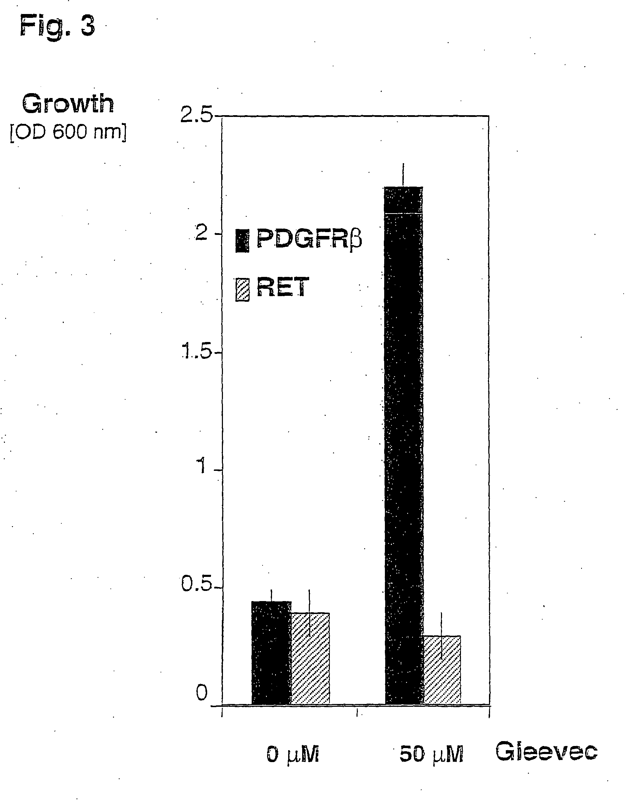

In an exemplary embodiment of the present invention, administration of the specific tyrosine kinase inhibitor imatinib mesylate (Gleevec® , Novartis) to engineered yeast cells expressing a dimerizing fragment of the RTK PDGFRβ was shown to block the dimerization-dependent kinase activity of this RTK and to restore growth under selective conditions (Figure 3). Growth inhibition caused by a different RTK (e.g. RET), which is known to be unaffected by the specific kinase inhibitor Gleevec, was not relieved in the presence of this compound (Figure 3).

Since bacterial and yeast cells do not have endogenous mammalian-type tyrosine kinases, this cell-based system offers the advantage of a null background for the expression of RTKs and for the screening of specific inhibitors of these membrane-bound kinases, a privileged situation that could not be obtained with mammalian cells.

A polypeptide of the present invention can be expressed from an extrachromosomal gene construct e.g. from an episomal vector enabling expression of the fusion protein in a host cell. The nucleic acid construct encoding the fusion peptide can be integrated into the genome of the host cell. The nucleic acid can be introduced into the cell by any transfection method leading to uptake of the nucleic acid sequence into the cell. Such methods are known to a person skilled in the art and are e.g described in Sambrook et al., Molecular Cloning: A Laboratory Manual, New York: Cold Spring Harbor Laboratory, 2001).

The construction of suitable host cells and the other molecular biological reagents for the use in the present invention e.g. fusion peptide constructs can be done using standard molecular biology techniques as described e.g. in Sambrook et al., Molecular Cloning: A Laboratory Manual, New York: Cold Spring Harbor Laboratory, 2001).

It was found that different strains (different genetic backgrounds), in particular different yeast strains, respond with different levels of growth rate reduction as a consequence of the expression of given RTKs. Thus, similarly to the above addressed use of various promoters (different expression levels), various yeast strains carrying various mutations may be used for the expression of RTKs. The availability of different cells with different levels of growth rate reduction assists in choosing the best performing system for each RTK to be tested.

Presently preferred yeast strains for use in the screening of RTK inhibitors are:
**RLY07:**
   *MATα; ura-52; his3Δ200; leu2Δ1; trp1Δ63; lys2Δ385; Δyor1; Δsnq2; Δpdr5*
**RLY07Δerg6:**
   *MATα; ura-52; his3Δ200; leu2Δ1; trp1Δ63; lys2Δ385; Δyor1; Δsnq2; Δpdr5; Δerg6*
**RLY09:**
   *MATα; pdr1-3; Δura3; Δhis1; Δyor1; Δsnq2; pdr5-Δ2 ; Δpdr10; Δpdr11; Δycf1; pdr3-Δ2; Δpdr15 ; Δleu2; Δtrp1*
**RLY07Δptp1:**
   *MATα; ura-52; his3Δ200; leu2Δ1; trp1Δ63; lys2Δ385; Δyor1; Δsnq2; Δpdr5; Δptp1*
**RLY07Δerg6Δptp1:**
   *MATα; ura-52; his3Δ200; leu2Δ1; trp1Δ63; lys2Δ385; Δyor1; Δsnq2; Δpdr5; Δerg6; Δptp1*
**RLY09Δptp1 :**
   *MATα; pdr1-3; Δura3; Δhis1; Δyor1; Δsnq2; pdr5-Δ2; Δpdr10; Δpdr11; Δycf1; pdr3-Δ2; Δpdr15 ; Δleu2; Δtrp1 ; Δptp1*

The person skilled in the art is as well able to determine suitable culturing conditions allowing the detection and/or survival of the used cells. Said conditions are dependent on the used genetic constructs and the host cells.

There are at least three different categories of compounds that can be screened by a screening method of the present invention: chemical libraries, natural product libraries and combinatorial libraries. Chemical libraries consist of structural analogues of known compounds. Natural product libraries are collections of microorganism, animals, plants or fungi which are used to create mixtures for screening by for example fermentation and extraction of broths from soil, or extraction of plants or marine organisms. Combinatorial libraries are composed of large numbers of peptides, oligonucleotides or organic compounds as a mixture. They are relatively easy to prepare e.g. by traditional synthesis methods, PCR or cloning.

As soon as the RTK with abnormal activity has been identified, it may be sequenced and the sequence entered into a data-base. Said data-base may e.g. comprise information on the sequence or the specific mutations, respectively, the activity, characteristics of the origin, e.g. the tumor, and on the inhibitors tested with indication of their performance, i.e. the responsiveness of a particular mutation to said inhibitor, epidemiological date etc. Such data-base then allows to faster characterize a tumor and to faster select the most promising therapy.

The invention is now further described by means of examples:

### Experiment 1: Suitability of different RTK fragments

The results of this experiment are shown in Figure 1 and Figures 2a and 2b.

Cell growth and colony formation on glucose plates, on which expression of the RTK genes under the control of the GAL-1 promoter (pMH3) is repressed, is shown on the left side. Cell growth and colony formation on galactose plates, on which RTK gene expression is induced, is shown on the right side. To constitutively activate c-Met and PDGFRβ, a heterologous dimerization domain was fused to the c-Met and PDGFRβ cytoplasmic domains or the c-Met and PDGFRβ longer derivatives that included the natural transmembrane domain. The c-Jun leucine zipper or the Fos leucine zipper were fused in frame to either the N-terminus of the cytoplasmic domains of c-Met (AA932-1366) and PDGFRβ (AA524-1067), or to the N-terminus of the c-Met (AA905-1366) and PDGFRβ (AA496-1067) constructs bearing the transmembrane domains.

Expression of active cytoplasmic domains of the RTKs c-Met and PDGFRβ causes kinase-dependent growth inhibition of yeast cells (Figure 1a and 1b, lanes 1 and 4). Inactivation of the kinase activity through mutation of a conserved lysine residue (Lys=K) in the ATP-binding pockets of these RTKs suppresses the growth inhibitory effect (Figure 1a and 1b, lanes 3 and 6). In contrast to the isolated cytoplasmic domains of these RTKs, inclusion of the respective transmembrane domains of these proteins, in addition to a signal peptide for ER localization and extra-cellular localization of the N-terminal domain, caused the abolition of the inhibitory effect on cell growth (Figure 1a and 1b, lanes 2 and 5).

To analyze expression of the constructs in yeast cells, an HA or a Myc epitope was inserted between the fusion site of the dimerization domain and kinase domain. For the cytoplasmic constructs, membrane localization was conferred by a myristolation signal (black bar anchored at the membrane). To ensure proper secretion of the fusion proteins containing their transmembrane domains, the yeast Suc2 signal sequence (SS) was fused N-terminally of the dimerization domains.

Expression of the indicated hybrid proteins was monitored by SDS-PAGE followed by Western blotting with anti-Myc antibodies (Figure 2a, two exposures shown). The indicated hybrid proteins, which carry the Myc epitope for detection, the Fos leucine zipper for heterodimer formation, and the myristolation signal (Myr) for membrane anchoring, were all expressed together with similar constructs carrying the HA epitope and the partner Jun leucine zipper instead of the functionally equivalent sequences of the detected proteins. Phosphorylation of proteins (autophosphorylation of the RTKs as well as transphosphorylation of uncharacterized substrate proteins) was detected by Western blotting with anti-pTyr antibodies (Figure 2b). Although c-Met and PDGFRβ derivatives bearing the transmembrane domains (TM) are active to an extent that is comparable to that of the isolated cytoplasmic domains of these RTKs (compare lane 1 with 2, and lane 4 with 5 of Figure 2b), they do not inhibit yeast proliferation. As expected, the inactivated kinase mutants did not show any tyrosine phosphorylation (Figure 2b, lanes 3 and 6).

### Experiment 2: Identification of RTK inhibitors

The results of this experiment are shown in Figure 3.

Selection of specific inhibition of Receptor Tyrosine Kinase activity in yeast. Expression of the human receptor tyrosine kinases PDGFR-β and RET in yeast causes strong retardation of cell growth, as determined by OD 600 nm light scattering measurement of both cell cultures. Addition of the kinase inhibitor imatinib mesylate (Gleevec® , Novartis), which is known to inhibit PDGFR-β but not RET, at a concentration of 50 µM in the yeast culture specifically restores growth of PDGFR-β-expressing yeast cells but not growth of cells expressing RET.

### Experiment 3: Different expression levels

EphB4 or RET were cloned under various GAL promoters which produce various levels of expression of the kinase (see Figures 4 to 6)and introduced into yeast. These promoters were called "100%" for the full-length promoter, and "70%". "50%", "17%" for truncated promoters which induce expression levels at the corresponding percentages of the full-length promoter.

A liquid growth assay was performed, using three different starting ODs, and growth of the various strains was measured.

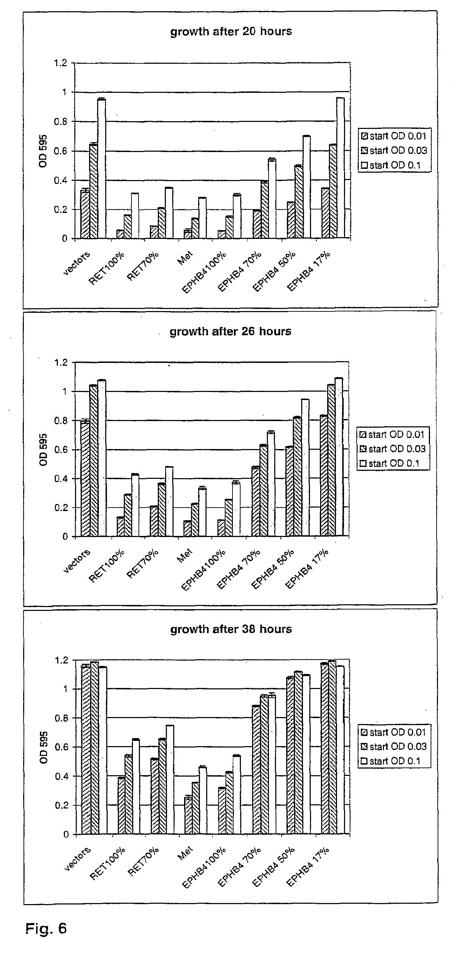

By this experiment it could be shown that when the expression levels of the RTKs EphB4 or RET are diminished in comparison to the expression level from the full-length GAL promoter, the slow growth phenotype of the kinase is reduced and growth is higher than for the "100%" strains (Figure 6).

### Experiment 4: KIT mutants in yeast

Mutations in Kit or autocrine/paracrine activation mechanisms of its pathway have been implicated in a variety of malignancies. For example, Kit is frequently mutated and activated in gastrointestinal stromal tumors (GISTs). Kit is a convenient target in Kit-induced tumors. In GISTs, about 50% of Kit mutations occur in exon 11, in the juxtamembrane (JM) region, including point mutations, insertions, or deletions.

STI571 (Gleevec) is very efficient in inhibiting this receptor; it was effective in treatment of GISTs where 80% of the patients showed a response. However a small proportion of patients showed resistance to the treatment (25)(30).

Mast cells retain Kit expression after differentiation. The Kit signaling pathway regulates mast cell function like degranulation and chemotaxis. Mastocytosis is characterized by an unusual expansion of the mast cell population and can progress to a mast cell leukemia. In adult mastocytosis, nearly all patients have a mutation in the active site at codon 816 (23). The D816V activating mutation is the most common and confers resistance to STI571 or SU9529 (29). Other mutations, like D816Y and D816F, which are rare, retain some sensitivity to Kit inhibitors (29).

Kit mutations are also found in t(8;21) AML (acute myeloid leukemia), the most frequent one being N822K, in the enzymatic pocket (38). The C-KIT mutations are closely correlated with t(8;21) leukemia. The fusion AE (AML1-ETO) is probably a very early event in leukemogenesis, but clones with AE alone might have very limited proliferative potential. The addition of mC-KIT as a secondary oncogenic event based on t(8;21) may accelerate the disease process. In addition, AE induces up-regulation of C-KIT, at least partially through down-regulation of TGF-β1.

Both types of mutations, in the juxtamembrane domain or in the phosphotransferase domain, induce ligand-independent activation of Kit; however it seems that the mechanisms underlying constitutive activation are distinct, because V559G undergoes dimerization but not D814V (26). Nevertheless, both induce a factor-independent and tumorigenic phenotype.

Such mutants were used to investigate the suitability of the yeast system of the present invention.

The most common mutations of Kit were analyzed in the above described yeast cell-based assay for RTKs. The mutations analyzed in the scope of the present invention were the following:
a) At position 816: this mutation occurs in mastocytosis, it is located in the activation loop and affects the structure of the catalytic site, it confers constitutive activation. D816 is critical to maintaining an inactive conformation of the activation loop. Mutation may shift the equilibrium to an open, ATP-binding conformation. The D816V mutation leads to ligand-independent activation, increased catalytic activity of Kit, and alters substrate specificity leading to aberrant signaling. This mutant is resistant to Gleevec, SU9529, and PD180970.
b) One of the mutations in the negative regulatory juxtamembrane domain, which are called regulatory type mutations because they are involved in the regulation of the otherwise normal catalytic site. These are the mutations found in GISTs. Kit mutant V560G (exon 11)is inhibited in vitro, similarly to wild-type Kit, by Gleevec and SU9529 (0.1-1 uM)(29). Both inhibitors induce apoptosis in the human cell line HMC1.1 (subclone of human mast cell leukemia line HMC1, expressing V560G mutation (29)). PD180970 inhibits cell proliferation and KIT phosphorylation in HMC-1 cells (23) .
c) K642E (in the TK1 tyrosine kinase domain, exon 13) and N822H (in the TK2 tyrosine kinase domain, exon 17): both mutations are found in GISTs but rarely; they are sensitive to Gleevec in vitro like wt KIT (100-200 nM; (25)).

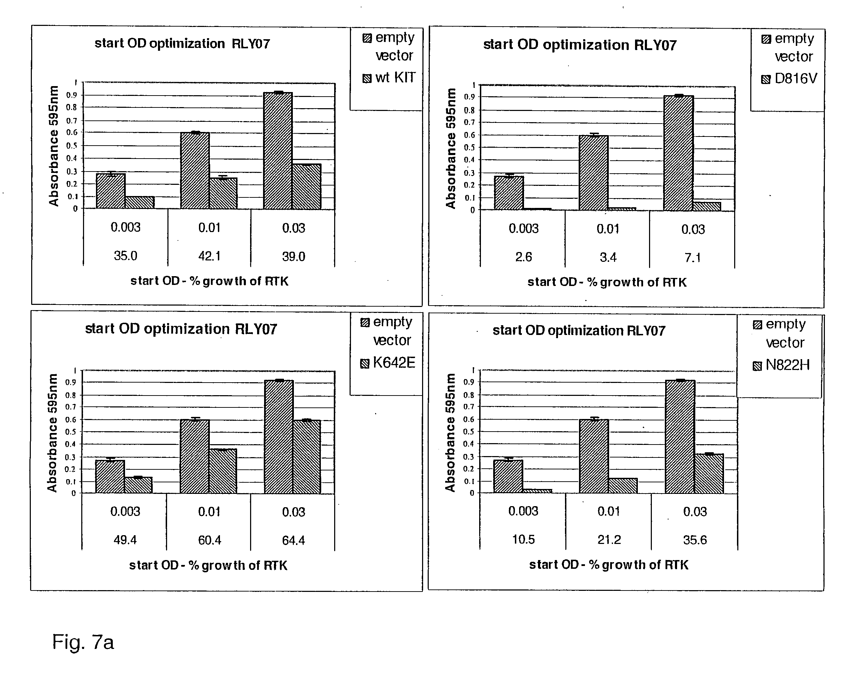

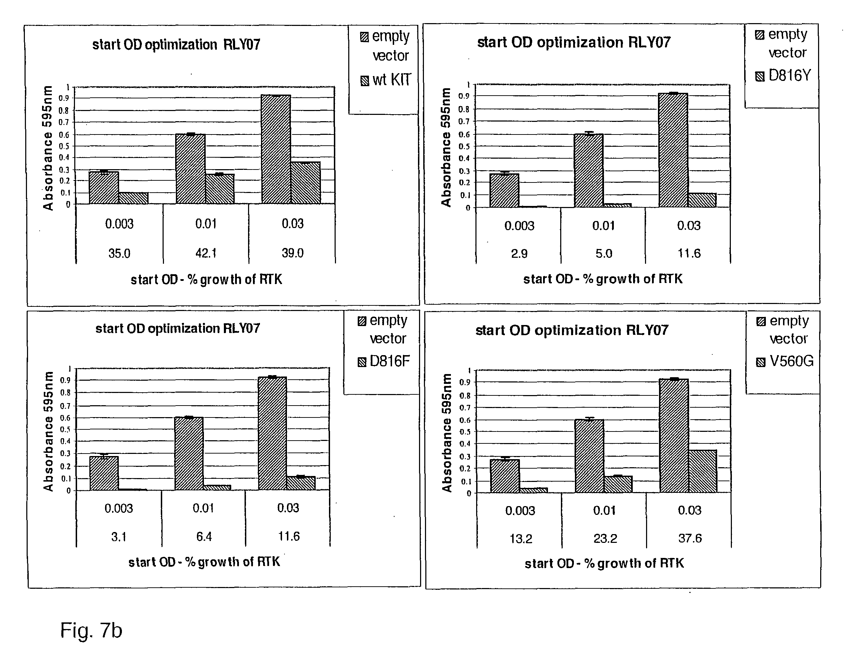

Expression of wild-type KIT confers only a slight slow growth phenotype in yeast (in the RLY07 strain, under the full-length GAL promoter). Various mutations that occur in human GISTs or in mastocytosis were introduced in the Kit construct expressed in yeast. Expression of the various mutants caused reduced yeast growth but in various extent, likely correlating with various extent of activity (see graphs Figures 7a and 7b) .

According to (27), a point mutation in the JM domain (BR canine mastocytoma cell line L575P, exon 11) leads to slightly less autophosphorylation of Kit than D816Y (P815, mouse mastocytoma cell line) and has a lower level of PI 3-kinase associated. There is a similar observation in (28). Interestingly, in yeast, a stronger reduction of growth was observed with the D816 mutants as with the juxtamembrane mutant V560G (graphs Figure 7a, 7b).

Three compounds (Gleevec, SU9529, and PD180970), at concentrations 0.2 to 100 µM (100, 50, 16.6, 5.5, 1.85, 0.62 and 0.21 µM) were tested for their ability to block wild-type Kit or mutant Kit in the yeast system. The yeast strain expressing the various Kit proteins in this experiment, was RLY09/Δptp1, in which all the Kit proteins produce a similar slow growth phenotype (see values of the window vector/RTK). The starting OD was optimized to obtain windows as similar as possible.

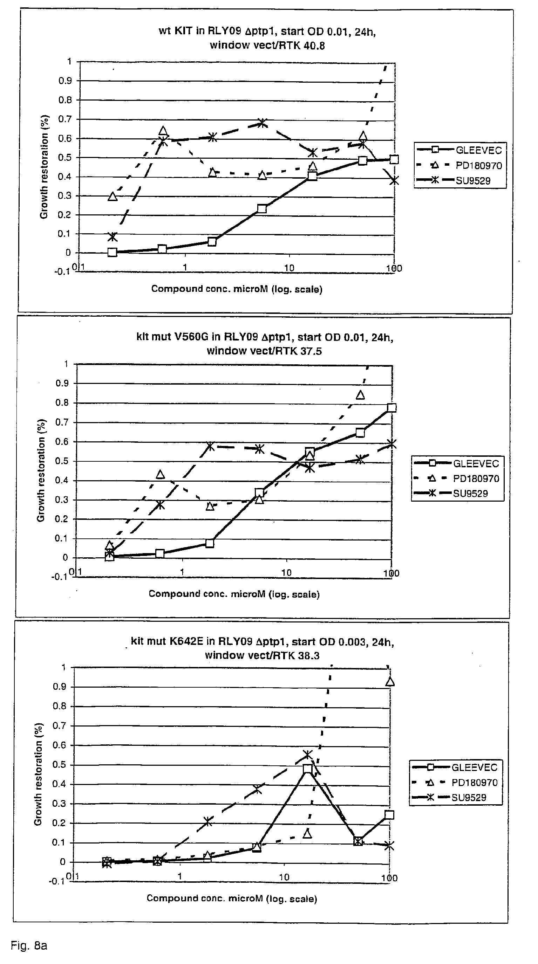

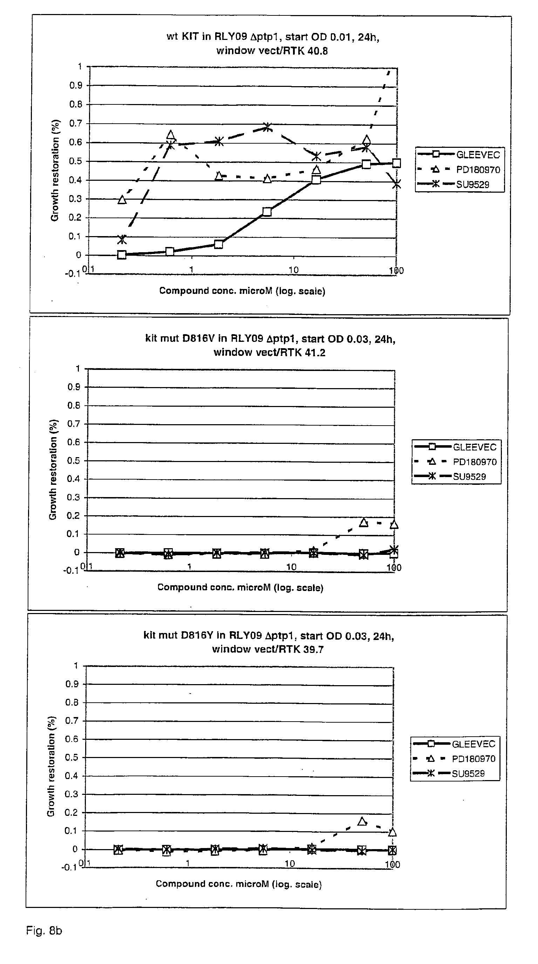

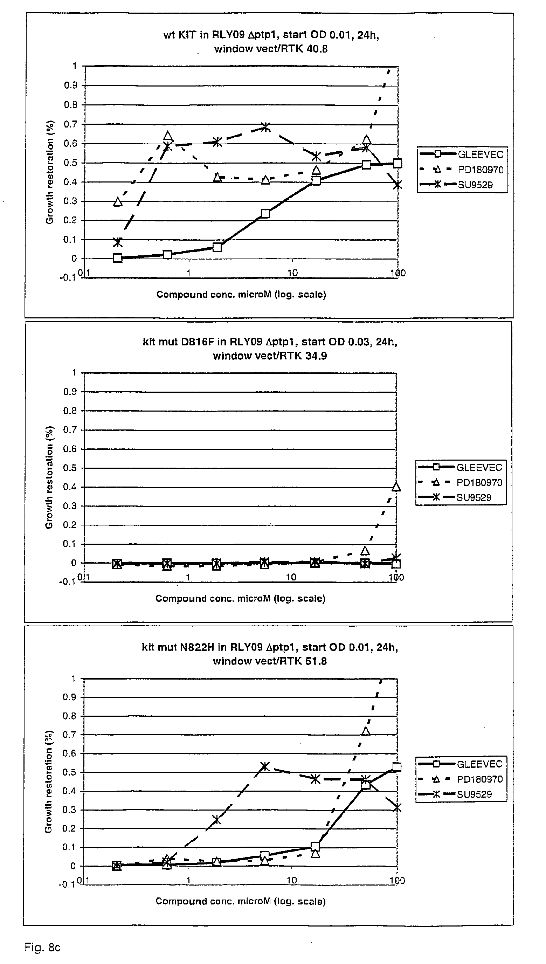

The results are presented in the graphs in Figures 8a to 8c) :

All three inhibitors rescued growth of the yeast strain expressing wild-type KIT (first panel). These inhibitors were also efficient against V560G, K642E, and N822H. On the contrary, the mutants D816V, D816F, and D816Y, were resistant to Gleevec and SU9529; PD180970 had a slight effect on these mutants at the highest concentrations. In the yeast strains used herein, the three different D816 mutants showed the same resistance towards the inhibitors tested. Overall the results presented here, in yeast, correlate with *in vitro*, *in vivo*, and clinical data concerning the activity of the various KIT mutants as well as their sensitivity to inhibitors.

### Experiment 5: EGFR mutants in Yeast

RTKs of the EGFR family regulate essential cellular functions, including proliferation, survival, migration and differentiation, and appear to play a central role in the etiology and progression of solid tumors (31, 32). EGFR itself is frequently overexpressed in lung, breast, colon, ovarian and brain tumors, prompting the development of specific tyrosine kinase inhibitors such as gefitinib (Iressa), which disrupts EGFR kinase activity by binding the ATP pocket within the catalytic domain (36). Gefitinib was approved for the treatment of nonsmall cell lung cancer (31, 32). However, most patients with nonsmall cell lung cancer have no response to gefitinib. Only 10% of patients have a rapid and often dramatic clinical response. It was recently shown that the presence of specific mutations in the EGFR tyrosine kinase in the vicinity of the ATP site correlates with sensitivity of nonsmall cell lung cancer to gefitinib. This discovery at least partially explains the clinical observation that only a subset of patients respond to treatment with gefitinib and provides a means to identify those patients most likely to benefit from the drug.

Somatic mutations that were identified in patients with gefitinib-responsive lung cancer were either small in-frame deletions or amino acid substitutions clustered around the ATP-binding pocket of the tyrosine kinase domain. In vitro, EGFR mutants demonstrated enhanced tyrosine kinase activity in response to EGF and increased sensitivity to inhibition by gefitinib (33, 20, 35). More recently, Pao and collaborators have shown that tumors sensitive to a related EGFR kinase inhibitor, erlotinib (Tarceva), contain similar types of EGFR mutations (34). Thus, similar to results with gefitinib, mutations in the EGFR TK domain are also associated with sensitivity of nonsmall cell lung cancer to erlotinib.

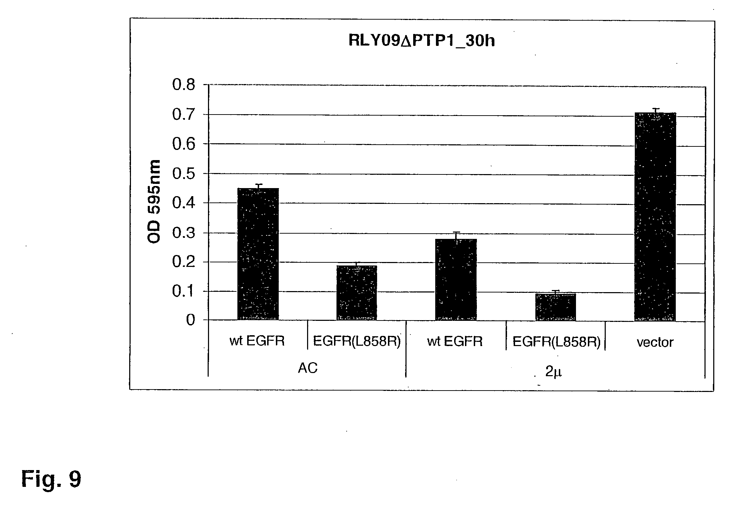

In the scope of the present invention, the two most common EGFR mutations were analyzed in the yeast cell-based assay for human RTKs: the missense mutation Leu⁸⁵⁸-Arg⁸⁵⁸ (L858R) and the 18-base pair in frame deletion (Del747-753insS). *S. cerevisiae* lacking PTP1 was transformed with low-copy (AC) or high copy (2µ) plasmids expressing the indicated EGFR derivatives under the control of the *GAL1* promoter. The cultures were grown in galactose medium to induce expression of the EGFR derivatives and growth was determined after 30 hours by OD₅₉₅ measurement. Expression of EGFR(L858R) that is reported to have enhanced kinase activity causes a kinase-dependent growth inhibition of yeast cells. Expression of wild-type EGFR only slightly inhibits growth in yeast, whereas the mutant shows significantly reduced growth of yeast cells as compared to yeast cells expressing the empty vector only (see Figure 9).

To test whether the severity of the growth is proportional to the level of EGFR activity, tyrosine phosphorylation in yeast cells was quantified by western blot analysis using anti-phosphotyrosine antibodies. Expression of the EGFR hybrid proteins was monitored with anti-Myc antibodies. In particular, expression of the indicated Myc-tagged EGFR mutants was monitored by SDS-PAGE followed by Western blotting with an anti-Myc antibody (top). Phosphorylation of proteins (autophosphorylation of the RTKs as well as transphosphorylation of uncharacterized yeast proteins) was detected by Western blotting with an anti-phosphotyrosine antibody (middle). An actin antibody was used to monitor the amounts of protein load for the extracts expressing the different EGFR mutants (bottom). Yeast cultures were grown in glucose medium overnight and then switched to the inducing galactose medium and grown to an OD₆₀₀ of 1. All the EGFR mutants were expressed to about the same level as wild-type EGFR.

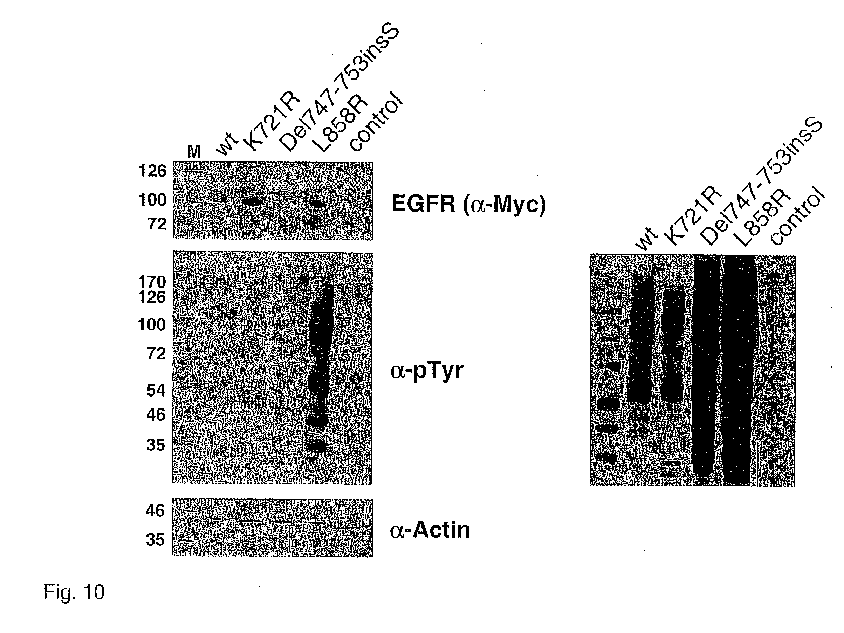

It was found that extracts of yeast cells expressing EGFR(L858R) displayed the highest level of tyrosine phosphorylation, namely a higher level of tyrosine phosphorylation compared to yeast cells expressing wild-type EGFR (Figure 10), such that the intensitiy of the phosphotyrosine profile of EGFR(L858R) expressing yeast cells parallels the strength of the 'growth inhibition' phenotype (see Figure 9). The deletion mutant EGFR(De1747-753insS) also showed enhanced tyrosine phosphorylation in comparison to wild-type EGFR, even though it did not confer a slow growth phenotype in yeast (Figure 10 and data not shown). The kinase-dead EGFR(K721R) showed decreased tyrosine phosphorylation.

To summarize, similar to the activity found in mammalian cells, the EGFR mutant EGFR(L858R) displayed enhanced tyrosine kinase activity in yeast as well.

While there are shown and described presently preferred embodiments of the invention, it is to be distinctly understood that the invention is not limited thereto but may be otherwise variously embodied and practiced within the scope of the following claims.

### References

1. Ullrich and J. Schlessinger, Signal transduction by receptors with tyrosine kinase activity. Cell 61 (1990), pp. 203-212.
2. S.C. Robertson et al., RTK mutations and human syndromes when good receptors turn bad. Trends Genet. 16 (2000), pp. 265-271.
3. D.J. Slamon et al., Human breast cancer: correlation of relapse and survival with amplification of the HER2/neu oncogene. Science 235 (1987), pp. 77-82.
4. S. Paik et al., Pathologic findings from the national surgical adjuvant breast and bowel project: Prognostic significance of erbB-2 protein overexpression in primary breast cancer. J. Clin. Oncol. 8 (1990), pp. 103-112.
5. Ullrich et al., Human epidermal growth factor receptor cDNA sequence and aberrant expression of the amplified gene in A431 epidermoid carcinoma cells. Nature 309 (1984), pp. 418-425.
6. W.K. Hong and A. Ullrich , The role of EGFR in solid tumors and implications for therapy. Oncol. Biother. 1 (2000), pp. 1-29.
7. T.A. Libermann et al., Amplification, enhanced expression and possible rearrangement of EGF receptor gene in primary human brain tumours of glial origin. Nature 313 (1985), pp. 144-147.
8. Pavelic et al., Evidence for a role of EGF receptor in the progression of human lung carcinoma. Anticancer Res. 13 (1993), pp. 1133-1138.
9. Levitzki , Protein tyrosine kinase inhibitors as novel therapeutic agents. Pharmacol. Ther. 82 (1999), pp. 231-239.
10. S.B. Noonberg and C.C. Benz, Tyrosine kinase inhibitors targeted to the epidermal growth factor receptor subfamily: Role as anticancer agents. Drugs 59 (2000), pp. 753-767.
11. R.P. Hertzberg, and A.J. Pope, High-throughput screening: new technology for the 21st century. Curr Opin Chem Biol 4 (2000), pp. 445-451.
12. Johnston, P. A. (2002) Cellular platforms for HTS: three case studies. Drug Discov Today 7, pp. 353-363.
13. Gonzalez, J. E., and Negulescu, P. A. (1998) Intracellular detection assays for high-throughput screening. Curr Opin Biotechnol 9, pp. 624-631.
14. Hughes, T. R. (2002) Yeast and drug discovery. Funct Integr Genomics 2, pp. 199-211.
15. Botstein, D., Chervitz, S. A., and Cherry, J. M. (1997) Yeast as a model organism. Science 277, pp. 1259-1260.
16. Munder, T., and Hinnen, A. (1999) Yeast cells as tools for target-oriented screening. Appl Microbiol Biotechnol 52, pp. 311-320.
17. Brenner, C. (2000) A cultivated taste for yeast. Genome Biol 1, Reviews 103.
18. Superti-Furga G, Jonsson K, Courtneidge S. A. (1996) A functional screen for regulators and antagonizers of heterologous protein tyrosine kinases. Nat Biotechnol 14(5), pp. 600-5.
19. Florio M., Wilson L. K., Trager J. B., Thorner J., Martin G. S. (1994) Aberrant protein phosphorylation at tyrosine in responsible for the growth-inhibitory action of pp60v-src expressed in the yeast Saccharomyces cerevisiae. Mol Biol Cell 5(3) pp. 283-96.
20. Paez J. G. et al. (2004) EGFR Mutations in Lung Cancer: Correlation with Clinical Response to Gefitinib Therapy. Science 304, pp. 1497-1500.
21. Lynch T. J. et al. (2004) Activating Mutations in the Epidermal Growth Factor Receptor Underlying Responsiveness of Non-Small-Cell Lung Cancer to Gefitinib. The New England Journal of Medicine 350(21) pp. LYNCH-1 - LYNCH-11.
22. Green M. R. (2004) Targeting Targeted Therapy. The New England Journal of Medicine 350(21), pp. 2191-2193.
23. Corbin, A. S., I. J. Griswold, et al. (2004). "Sensitivity of oncogenic KIT mutants to the kinase inhibitors MLN518 and PD180970." Blood 10, pp. 10.
24. Gunde T. (2004) In Vivo Veritas? Cell-Based Assay for Identifying RTK Inhibitors. European BioPharmaceutical Review, Spring issue.
25. Heinrich, M. C., C. L. Corless, et al. (2003). Kinase mutations and imatinib response in patients with metastatic gastrointestinal stromal tumor. J Clin Oncol 21(23), pp. 4342-4349.
26. Kitayama, H. and e. al. (1995). Constitutively activating mutations of c-kit receptor tyrosine kinase confer factor-independent growth and tumorigenicity of factor-dependent hematopoietic cell lines. Blood 85(3), pp. 790-798.
27. Liao, A. T., M. B. Chien, et al. (2002). Inhibition of constitutively active forms of mutant kit by multitargeted indolinone tyrosine kinase inhibitors. Blood 100(2), pp. 585-593.
28. Ma, Y., E. Carter, et al. (2000). Indolinone derivatives inhibit constitutively activated KIT mutants and kill neoplastic mast cells. J Invest Dermatol 114(2), pp. 392-394.
29. Ma, Y., S. Zeng, et al. (2002). The c-KIT mutation causing human mastocytosis is resistant to STI571 and other KIT kinase inhibitors; kinases with enzymatic site mutations show different inhibitor sensitivity profiles than wild-type kinases and those with regulatory-type mutations. Blood 99(5), pp. 1741-1744.
30. Sattler, M. and R. Salgia (2004). Targeting c-Kit mutations: basic science to novel therapies. Leukemia Research 2851(S1), pp. S11-S20.
31. Levitzki, A. (2003). EGF receptor as a therapeutic target. Lung Cancer 41(Suppl 1), pp. S9-S14.
32. Levitzki, A. (2003). Protein kinase inhibitors as a therapeutic modality. Acc Chem Res 36(6), pp. 462-469.
33. Lynch, T. J., D. W. Bell, et al. (2004). Activating mutations in the epidermal growth factor receptor underlying responsiveness of non-small-cell lung cancer to gefitinib. N Engl J Med 350(21), pp. 2129-39. Epub 2004 Apr 29.
34. Pao, W., V. Miller, et al. (2004). EGF receptor gene mutations are common in lung cancers from "never smokers" and are associated with sensitivity of tumors to gefitinib and erlotinib. Proc Natl Acad Sci U S A 101(36), pp. 13306-11. Epub 2004 Aug 25.
35. Sordella, R., D. W. Bell, et al. (2004). Gefitinib-sensitizing EGFR mutations in lung cancer activate anti-apoptotic pathways. Science 305(5687), pp. 1163-7. Epub 2004 Jul 29.
36. Wakeling, A. E., S. P. Guy, et al. (2002). ZD1839 (Iressa): an orally active inhibitor of epidermal growth factor signaling with potential for cancer therapy. Cancer Res 62(20), pp. 5749-54.
37. Liang, S.D. et al. (1996). DNA Sequence Preferences of GAL4 and PRP1: How a Subset of Zn2Cys6 Binuclear Cluster Proteins Recognizes DNA. Molecular and Cell Biology 16(7), pp. 3773-3780
38. Wang, Y.-Y., G.-B. Zhou, et al. (2005). "AML1-ETO and C-KIT mutation/overexpression in t(8;21) leukemia: Implication in stepwise leukemogenesis and response to Gleevec." PNAS 102(4): 1104-1109.

## Claims

1. A method for the identification of mutations in a receptor tyrosine kinase leading to a change in activity, said method comprising the following steps:
a) providing a nucleic acid construct, said nucleic acid construct comprising under the control of an inducible promoter a nucleic acid sequence of a particular individual encoding a polypeptide which comprises a tyrosine kinase domain of a receptor tyrosine kinase or a fragment of said tyrosine kinase domain wherein said polypeptide lacks a transmembrane domain or a functional fragment of said transmembrane domain,
b) introducing said nucleic acid construct into a host cell and
c) culturing said cells under conditions in which the tyrosine kinase activity of an active RTK leads to proliferation reduction or proliferation arrest of said host cells,
d) determining the activity level of said RTK, and
e) comparing the activity of said RTK with the activity determined for a standard form of the respective RTK under the same conditions.

2. A method for the identification and/or validation of inhibitors of a specific receptor tyrosine kinase activity, said method comprising the following steps:
a) providing host cells comprising a nucleic acid construct of a particular individual tyrosine kinase activity under the control of an inducible promoter, said nucleic acid construct encoding a polypeptide which comprises a tyrosine kinase domain of a receptor tyrosine kinase or a functional fragment thereof wherein said polypeptide lacks a transmembrane domain or a functional fragment of said transmembrane domain and wherein said tyrosine kinase activity leads to proliferation reduction or arrest of said host cells,
b) contacting said host cells with at least one candidate compound and
c) determining the inhibitory effect of such at least one candidate compound on said tyrosine kinase activity by cultivation of said host cells under suitable conditions such that a modulation of the tyrosine kinase activity by said at least one candidate compound leads to cell proliferation or enhanced cell proliferation.

3. The method of claim 1 or 2, wherein said inducible promoter is a galactose inducible promoter, in particular a GAL1 promoter or promoter derived thereof with reduced protein expression ability.

4. The method of claim 3, wherein the inducible promoter is the GAL1 promoter.

5. The method of claim 3, wherein the inducible promoter is a promoter derived from the GAL1 promoter with reduced protein expression ability, in particular a promoter selected from those designated as pRL23, pRL29 or pRL17.

6. The method of any one of the preceding claims, wherein said nucleic acid construct encodes a polypeptide which comprises the entire cytoplasmic part of said receptor tyrosine kinase.

7. The method of any one of the preceding claims, wherein said polypeptide further comprises a dimerization domain or a functional fragment thereof.

8. The method of any one of the preceding claims, wherein said dimerization domain is selected from c-Fos leucine zipper, c-Jun leucine zipper and Gcn4 leucine zipper.

9. The method of claim 7 or 8, wherein said cells comprise two polypeptides wherein the first polypeptide comprises a c-Fos leucine zipper and the second polypeptide comprises a c-Jun leucine zipper.

10. The method of any one of the preceding claims, wherein said polypeptide further comprises a sequence leading to membrane anchoring of the polypeptide, in particular a myristolation signal or a farnesylation signal, most preferably a myristolation signal for membrane anchoring.

11. The method of any one of the preceding claims, wherein said receptor tyrosine kinase is selected from the group consisting of EGFR, ERBB2, ERBB3, ERBB4, INSR, IGF-1R, IRR, PDGFRα, PDGFRβ, CSF-1R, KIT/SCFR, FLK2/FLT3, VEGRF 1-3, FGFR 1-4, CCK4, TRKA, TRKB, TRKC, MET, RON, EPHA 1-8, EPHB 1-6, AXL, MER, TYRO3, TIE, TEK, RYK, DDR1, DDR2, RET, ROS, LTK, ALK, ROR1, ROR2, MUSK, AATYK, AATYK2, AATYK3, RTK106.

12. The method of claim 11, wherein said receptor tyrosine kinase is selected from the group consisting of INSR, IGF-1R, PDGFRα, PDGFRβ, KIT/SCFR, TRKA, MET, RON, EPHB2, EPHB4, AXL, TEK, RET, ROS, CSF-1R, FLK2/FLT3, TRKB, TRKC, EPHA 2, TIE, ALK, EGFR, ERBB2, VEGFR 1, VEGFR 2, FGFR 1-4.

13. The method of claims 11 or 12, wherein said receptor tyrosine kinase has human origin.

14. The method of any one of the preceding claims, wherein said host cells are yeast cells.

15. The method of claim 14, wherein said cells are S. cerevisiae cells, more preferably S. cerevisiae cells harboring a mutation in one gene or any combination of genes selected from the group comprising the ABC transporter genes, the ERG6 gene and genes encoding phosphatases, in particular the PTP1 gene.

16. The method of claim 15, wherein said cells are selected from
**RLY07:**
*MATα; ura-52; his3Δ200; leu2Δ1; trp1Δ63; lys2Δ385; Δyor1; Δsnq2; Δpdr5*
**RLY07Δerg6:**
*MATα; ura-52; his3Δ200; leu2Δ1; trp1Δ63; lys2Δ385; Δyor1; Δsnq2; Δpdr5; Δerg6*
**RLY09:**
*MATα; pdrl -3 ; Δura3; Δhis1; Δyor1; Δsnq2; pdr5-Δ2; Δpdr10; Δpdr11; Δycf1; pdr3-Δ2; Δpdr15; Δleu2; Δtrp1*
**RLY07Δptp1:**
*MATα; ura-52; his3Δ200; leu2Δ1; trp1Δ63; lys2Δ385; Δyor1; Δsnq2; Δpdr5; Δptp1*
**RLY07Δerg6Δptp1:**
*MATα; ura-52; his3Δ200; leu2Δ1; trp1Δ63; lys2Δ385; Δyor1; Δsnq2; Δpdr5; Δerg6; Δptp1*
**RLY09Δptp1:**
*MATα; pdr1-3; Δura3; Δhis1; Δyor1; Δsnq2; pdr5-Δ2; Δpdr10; Δpdr11; Δycf1; pdr3-Δ2; Δpdr15; Δleu2; Δtrp1; Δptp*1

17. The method of any one of the preceding claims, wherein said polypeptide lacks a signal sequence and/or peptide leading to nuclear localisation of said polypeptide.

18. The method of anyone of the preceding claims, wherein the data generated are entered into a data-base, said data-base comprising sequence information and at least one further information selected from activity and responsiveness to specific inhibitors, and optionally tissue specific data, in particular including tumor type DNA profiling, and/or patient data and/or epidemiological data.

19. The method of anyone of claims 2 to 18, wherein the at least one candidate compound is comprised in a mixture of possible candidate compounds.

20. The method of anyone of claims 2 to 18, wherein the at least one candidate compound is one specific candidate compound.

21. The method of anyone of claims 2 to 18, wherein the at least one candidate compound is a member of a library of candidate compounds.

22. A yeast expression vector comprising a nucleic acid sequence under the control of an inducible promoter encoding a polypeptide which comprises a tyrosine kinase domain of a receptor tyrosine kinase or a functional fragment thereof wherein said receptor tyrosine kinase has abnormal activity compared to the wild-type receptor tyrosine kinase and wherein said receptor tyrosine kinase or a functional fragment thereof lacks a transmembrane domain or a functional fragment thereof and lacks a signal sequence and/or peptide leading to nuclear localization of said receptor tyrosine kinase.

23. The yeast expression vector of claim 22, wherein said polypeptide further comprises a dimerization domain or a functional fragment thereof.

24. The yeast expression vector of claim 23, wherein said dimerization domain is selected from c-Jun leucine zipper, c-Fos leucine zipper and Gcn4 leucine zipper.

25. A yeast cell comprising a yeast expression vector of anyone of claims 22 to 24, in particular a yeast cell as defined claim 15 or 16.

26. A kit for the identification and/or validation of inhibitors of a receptor tyrosine kinase comprising a yeast expression vector of any one of claims 22 to 24 and/or a yeast cell of claim 25.
